# EUROPEAN PATENT SPECIFICATION

(11) **EP 1 201 243 B1**
(45) Date of publication and mention of the grant of the patent: **03.09.2003**
(21) Application number: 00122331.2
(22) Date of filing: 23.10.2000
(51) Int. Cl.: A61K 31/635, A61P 15/10

(54) **Use of sulfadiazine and sulfadimidine for treating erectile dysfunction**
Verwendung von Sulfadiazin und Sulfadimidine zur Behandlung der erektilen Dysfunktion
Utilisation de la sulfadiazine et de la sulfadimidine pour traiter la dysfonction érectile

(43) Date of publication of application: 02.05.2002
(73) Proprietor: The Jordanian Pharmaceutical Manufacturing and Medical Equipment Co.Ltd., Jo-Naor 11710 (JO)
(72) Inventor: Badwan, Adnan A.,Dr., Amman 11185 P.O. Box 851674 Jordan (JO)
(74) Representative: Winkler, Andreas, Dr.

(56) References cited:
- WO-A-94/28902
- KUENZEL, W. J. (1) ET AL: "Early sexual maturation induced by sulfamethazine in chicks is mediated by elevated LH and FSH release and transient inhibition of thyroid hormones." POULTRY SCIENCE, (1995) VOL. 74, NO. SUPPL. 1, PP. 75. MEETING INFO.: EIGHTY-FOURTH ANNUAL MEETING OF THE POULTRY SCIENCE ASSOCIATION, INC. EDMONTON, ALBERTA, CANADA AUGUST 14-18, 1995 , XP000986400
- SCHLEGEL P.N. ET AL: "Antibiotics: Potential hazards to male fertility." FERTILITY AND STERILITY, (1991) 55/2 (235-242). , XP000981814

## Description

The invention is related to a new use of a compound of the general formula (I) 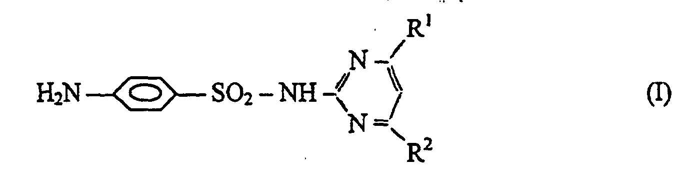 wherein R¹ and R² are independently selected from H and alkyl with from 1 to 4 carbon atoms, in particular a new use of sulfadiazine (R¹=R²=H) and sulfadimidine (R¹=R²=CH₃) and their pharmaceutically acceptable salts, in particular their sodium salt.

Sulfadiazine (N¹-(2-pyrimidinyl)sulphanilamide) is a well known therapeutic agent used for several indications, such as prophylaxis of recurrent rheuma, nocardiosis, asymptomatic meningococca, malaria, and toxoplasmosis (in patients with AIDS). Moreover, the silver salt of sulfadiazine is known as a wound disinfectant, e.g. for the treatment of burns.

The closely related compound sulfadimidine (N¹-(4,1-dimethyl-2-pyrimidinyl)sulphanilamide), also usually used in the form of its sodium salt, is another well known therapeutic agent with similar indications as sulfadiazine.

Surprisingly, it has now been found that compounds according to general formula (I), in particular sulfadiazine and sulfadimidine, have a significant improving effect on penile erection resulting in the new medical indication claimed in this application.

Thus, the present application is directed to the use of a compound of the general formula (I) 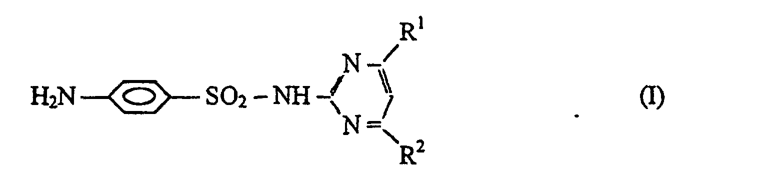 wherein R¹ and R² are independently selected from H and alkyl with from 1 to 4 carbon atoms, or a pharmaceutical acceptable salt thereof for the preparation of a medicament for the treatment of erectile dysfunction.

Preferred compounds are sulfadiazine, i.e. wherein both R¹ and R² are H, or sulfadimidine, i.e. wherein both R¹ and R² are CH₃, or pharmaceutically acceptable salts thereof.

The preferred dose per application is within a range of from 0.01 to 100 mg per kg body weight. The preferred way of administration is peroral so that the compound is preferably contained in an oral, nasal spray, sublingual or patches dosage form. However, any other dosage form may be suitable.

Surprisingly, the compounds of the formula (I) have shown a similar, if not better, Penile Erection Index than that of sildenafil (Viagra®).

The Penile Erection Index (PEI) is calculated or expressed as the % of rats exhibiting at least one episode of penile erection multiplied by the number of total episodes, within a time period of 2 hours. Details on the determination of PEI values can be found in e.g. H.H. Ang and M.K. Sim, Effects of Eurycoma longifilia Jack on Penile Erection Index and Homosexual Mounting in Rats, Pharmaceutical Sciences, 3 (1997), 117-19; and A. Benassi-Benelli, F. Ferrari and B. Pellegrini Quaratotti, Penile erection Induced by Apomorphine and N-n-propylnorapomorphine in Rats, Arch. Int. Pharmacodyn 242 (1979), 241-247.

The procedure, followed for the present invention, is as follows:

Wistar male rats were divided into 4 groups of each 10 rats. The first group, the control group, was given a placebo, namely distilled water as used for dissolving the active agent for the experimental groups. The other three groups, the experimental groups, were given different (oral by gavage) doses of the compound dissolved in above-mentioned vehicle. Each group was placed in a glass observation cage. All groups are done at the same time and observed in groups often at the same time. To confirm the activity, the experiments were repeated at least twice.

The dose dependent effects of sulfadiazine and sulfadimidine on the PEI can be seen from Table 1

**Table 1**

| Dose (mg/kg body weight) | Penile Erection Index (PEI) | |
|---|---|---|
| | sulfadiazine | sulfadimidine |
| 0.01 | 580 | - |
| 0.1 | 650 | 350 |
| 1 | 1.320 | 700 |
| 2 | 1.710 | 910 |
| 5 | 600 | 200 |
| 10 | 540 | 40 |
| 50 | 700 | 90 |
| 100 | 560 | - |

As can be seen from Table 1, sulfadiazine is significantly effective within the whole range of 0.01 to 100 mg per kg body weight per application, with a maximum activity between 0.1 and 5, in particular around 1 to 2 mg per kg body weight per application. For sulfadimidine, there is a significant activity in the range between 0.1 and 5 mg/kg body weight per application, the maximum being around 2 mg per kg body weight per application.

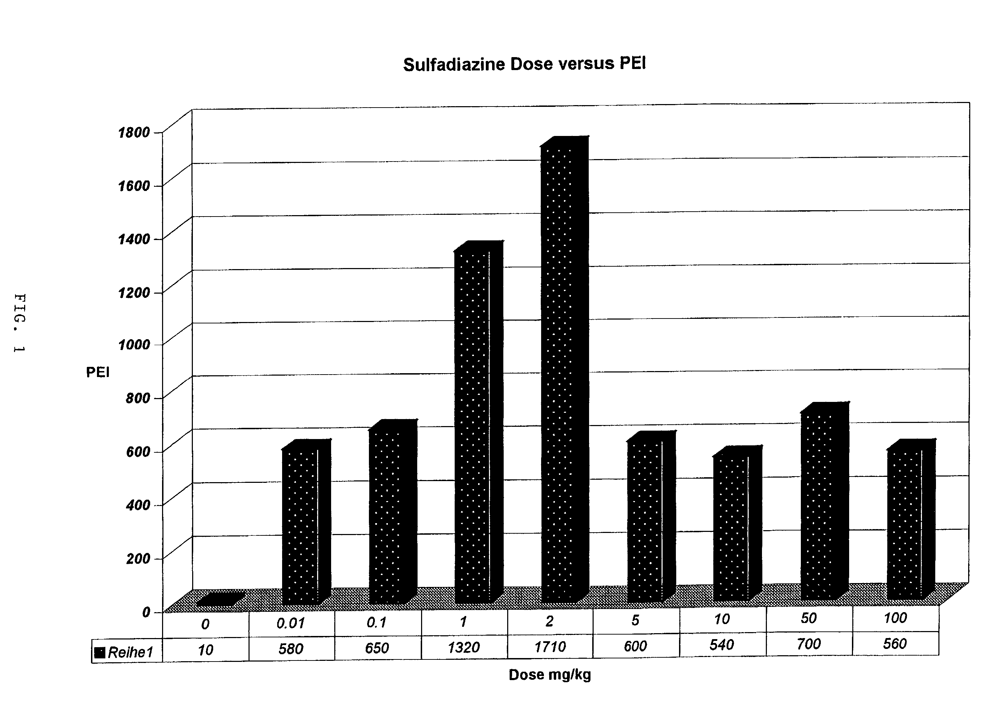

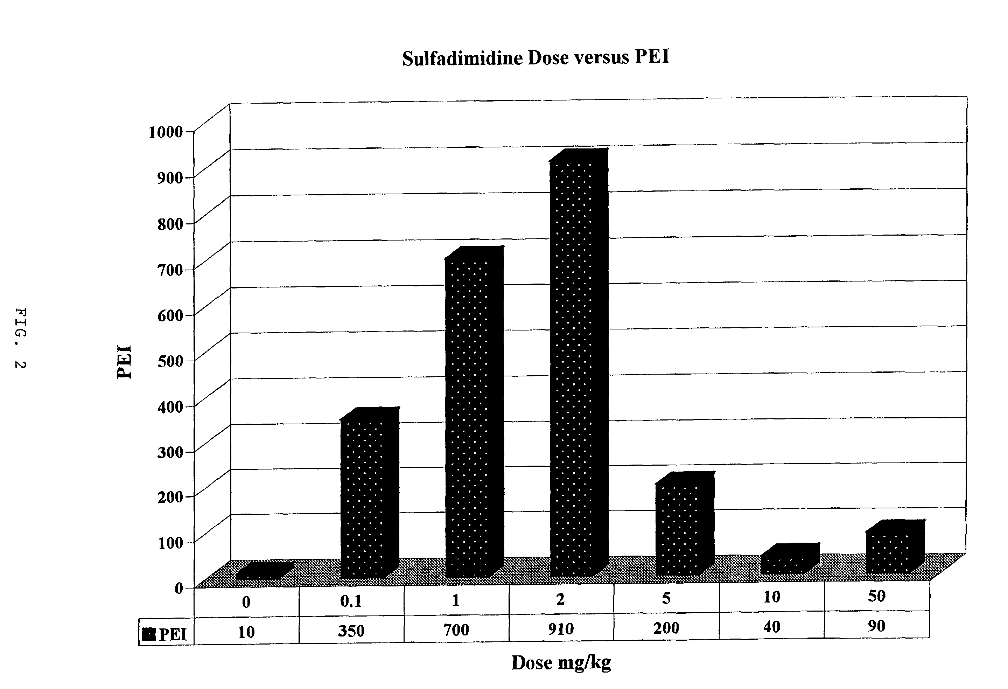

Graphic representations of the data of Table 1 are shown in Figs. 1 and 2.

A PEI as well as ED₅₀ comparison with sildenafil can be seen from Table 2.

**Table 2**

| Drug | Dose (mg/kg body weight) | PEI | PEI/Sildenafil PEI | Effective Dose ED₅₀ (mg/kg body weight) |
|---|---|---|---|---|
| sildenafil | 0.625 | 980 | 1.00 | 0.2843 |
| sulfadiazine | 2.0 | 1710 | 1.74 | 1.303 |
| sulfadimidine | 2.0 | 910 | 0.93 | 0.9026 |

From that comparison, it can be seen that sulfadiazine is superior and sulfadimidine at about equivalent to the well-known drug sildenafil.

In further experiments, sulfadiazine and sulfadimidine have shown to be able to relax contracted rabbit carvenosum corpora similar to sildenafil. Isolated corpora from rabbits penis were used. Relaxation was similar in magnitude as that for sildenafil.

The features disclosed in the foregoing description, in the claims and/or figures in the accompanying drawings may, both separately and in any combination thereof, be material for realizing the invention in diverse forms thereof.

## Claims

1. Use of a compound of the general formula (I) 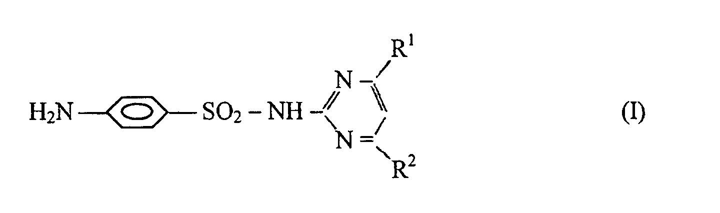 wherein R¹ and R² are independently selected from H and alkyl with from 1 to 4 carbon atoms, or a pharmaceutically acceptable salt thereof for the preparation of a medicament for the treatment of erectile dysfunction.

2. Use according to claim 1, wherein both R¹ and R² are H.

3. Use according to claim 1, wherein both R¹ and R² are CH₃.

4. Use according to one of claims 1 to 3, wherein the dose per application is within a range of from 0.01 to 100 mg per kg body weight.

5. Use according to one of the preceding claims, wherein the compound is contained in an oral, nasal spray, sublingual or patches dosage form.

## Patentansprüche

1. Verwendung einer Verbindung der allgemeinen Formel (I) 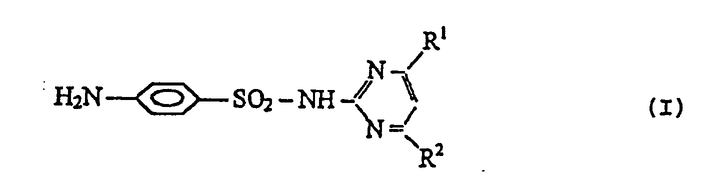 worin R¹ und R² unabhängig ausgewählt sind aus H und Alkyl mit von 1 bis 4 Kohlenstoffatomen, oder eines pharmazeutisch annehmbaren Salzes derselben zur Herstellung eines Arzneimittels zur Behandlung erektiler Dysfunktion.

2. Verwendung nach Anspruch 1, **dadurch gekennzeichnet, daß** sowohl R¹ als auch R² H sind.

3. Verwendung nach Anspruch 1, **dadurch gekennzeichnet, daß** sowohl R¹ als auch R² CH₃ sind.

4. Verwendung nach einem der Ansprüche 1 bis 3, **dadurch gekennzeichnet, daß** die Dosis pro Anwendung in einem Bereich von 0,01 bis 100 mg pro kg Körpergewicht liegt.

5. Verwendung nach einem der vorangehenden Ansprüche, **dadurch gekennzeichnet, daß** die Verbindung in einer oralen, Nasalspray-, sublingualen oder Pflaster-Dosierungsform enthalten ist.

## Revendications

1. Utilisation d'un composé de formule générale (I) 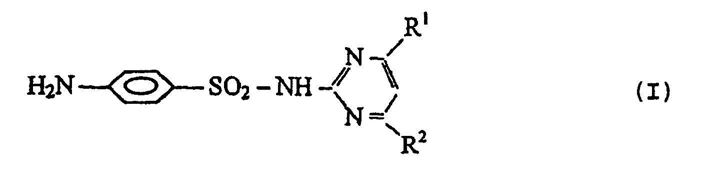 dans laquelle R¹ et R² sont, indépendamment l'un de l'autre, choisis parmi H et un groupe alkyle avec de 1 à 4 atomes de carbone, ou un de leurs sels acceptable sur le plan pharmaceutique pour la préparation d'un médicament destiné au traitement de la dysfonction érectile.

2. Utilisation selon la revendication 1, dans laquelle R¹ et R² représentent tous deux H.

3. Utilisation selon la revendication 1, dans laquelle R¹ et R² représentent tous deux CH₃.

4. Utilisation selon l'une quelconque des revendications 1 à 3, dans laquelle la dose par application est dans une gamme allant de 0,01 à 100 mg par kg de poids corporel.

5. Utilisation selon l'une quelconque des revendications précédentes, dans laquelle le composé est contenu dans une forme pharmaceutique orale, de pulvérisation nasale, sublinguale ou de timbres.
